# EUROPEAN PATENT SPECIFICATION

(11) **EP 1 043 395 B1**
(45) Date of publication and mention of the grant of the patent: **03.12.2008**
(21) Application number: 98961610.7
(22) Date of filing: 25.12.1998
(51) Int. Cl.: C07K 14/705, C12N 15/12, C12N 5/10, C12N 1/21, C12P 21/02, G01N 33/15, G01N 33/566, C12Q 1/02, C07K 16/28

(54) **NOVEL G PROTEIN-COUPLED RECEPTOR PROTEINS**
NEUE G-PROTEIN GEKOPPELTE REZEPTORPROTEINE
NOUVELLES RECEPTEURS LIES A LA PROTEINE G

(30) Priority: 26.12.1997 JP 36118797
(43) Date of publication of application: 11.10.2000
(73) Proprietor: BANYU PHARMACEUTICAL CO., LTD., Chuo-ku, Tokyo 103-8416 (JP)
(72) Inventor: ITADANI, Hiraku, Banyu Pharmaceutical Co., Ltd, Tsukuba-shi, Ibaraki 300-2611 (JP); TAKIMURA, Tetsuo, Banyu Pharmaceutical Co., Ltd, Tsukuba-shi, Ibaraki 300-2611 (JP); NAKAMURA, Takao, Banyu Pharmaceutical Co., Ltd, Tsukuba-shi, Ibaraki 300-2611 (JP); OHTA, Masataka, Banyu Pharmaceutical Co., Ltd, Tsukuba-shi, Ibaraki 300-2611 (JP)
(74) Representative: Vossius & Partner
(86) International application number: PCT/JP1998/005967
(87) International publication number: WO 1999/033978

(56) References cited:
- WO-A-00/20011
- WO-A-99/28470
- JP-A- 7 067 654
- DATABASE EMBL [Online] R87217 yo45e10.r1 Soares adult brain, 10 October 1995 (1995-10-10) HILLIER ET AL.: "The WashU-Merck EST Project" Database accession no. 946030 XP002196770
- LEURS R ET AL: "THE HISTAMINE H3-RECEPTOR: A TARGET FOR DEVELOPING NEW DRUGS" PROGRESS IN DRUG RESEARCH - FORTSCHRITTE DER ARZNEIMITTELFORSCHUNG - PROGRES DES RECHERCHES PHARMACEUTIQUES, BASEL, BIRKHAUSER VERLAG, CH, 1992, pages 127-165, XP000872231
- CHERIFI Y ET AL: "PURIFICATION OF A HISTAMINE H3 RECEPTOR NEGATIVELY COUPLED TO PHOSPHOINOSITIDE TURNOVER IN THE HUMAN GASTRIC CELL LINE HGT1" JOURNAL OF BIOLOGICAL CHEMISTRY, AMERICAN SOCIETY OF BIOLOGICAL CHEMISTS, BALTIMORE, MD, US, vol. 267, no. 35, 15 December 1992 (1992-12-15), pages 25315-25320, XP002916162 ISSN: 0021-9258
- ARRANG J-M ET AL: "AUTO INHIBITION OF BRAIN HISTAMINE RELEASE MEDIATED BY A NOVEL CLASS H-3 OF HISTAMINE RECEPTOR" NATURE (LONDON), vol. 302, no. 5911, 1983, pages 832-837, XP001068971 ISSN: 0028-0836
- BONNER T.I. et al., "Identification of a Family of Muscarinic Acetylcholine Receptor Genes", SCIENCE, (1987), Vol. 237, pages 527-532, XP002920349
- DEWAN ZENG et al., "Molecular Characterization of a Rat Alpha2B-Adrenergic Receptor", PROC. NATL. ACAD. SCI. USA, (1990), Vol. 87, No. 8, pages 3102-3106, XP002920350
- PERALTA E.G. et al., "Distinct Primary Structures, Ligand-Binding Properties and Tissue-Specific Expression of Four Human Muscarinic Acetylcholine Receptors", THE EMBO J., (1987), Vol. 6, No. 13, pages 3923-3929, XP002920351
- BRAUN T. et al., "A Novel Subtype of Muscarinic Receptor Identified by Homology Screening", BIOCHIM. BIOPHYS. RES. COMMUN., (1987), Vol. 149, No. 1, pages 125-132, XP002920352
- BONALDO M.F. et al., "Normalization and Subtraction: Two Approaches to Facilitate Gene Discovery", GENOME RES., (1996), Vol. 6, No. 9, pages 791-806, XP002920353

### SEQUENCE LISTING

<110> BANYU PHARMACEUTICAL CO., LTD.
<120> GUANOSINE TRIPHOSPHATE (GTP) BINDING PROTEIN-COUPLED RECEPTOR PROTEIN
<130> B1-902PCT
<140>
   <141>
<150> JP 1997-361187
   <151> 1997-12-26
<160> 24
<170> PatentIn Ver. 2.0
<210> 1
   <211> 413
   <212> PRT
   <213> Rattus norvegicus
<400> 1
   Met Glu Arg Ala Pro Pro Asp Gly Leu Met Asn Ala Ser Gly Thr Leu
   1               5                   10                  15
   Ala Gly Glu Ala Ala Ala Ala Gly Gly Ala Arg Gly Phe Ser Ala Ala
               20                  25                  30
   Trp Thr Ala Val Leu Ala Ala Leu Met Ala Leu Leu Ile Val Ala Thr
           35                  40                  45
   Val Leu Gly Asn Ala Leu Val Met Leu Ala Phe Val Ala Asp Ser Ser
       50                  55                  60
   Leu Arg Thr Gln Asn Asn Phe Phe Leu Leu Asn Leu Ala Ile Ser Asp
   65                  70                  75                  80
   Phe Leu Val Gly Ala Phe Cys Ile Pro Leu Tyr Val Pro Tyr Val Leu
   85 90 95
   Thr Gly Arg Trp Thr Phe Gly Arg Gly Leu Cys Lys Leu Trp Leu Val
   100 105 110
   Val Asp Tyr Leu Leu Cys Ala Ser Ser Val Phe Asn Ile Val Leu Ile
   115 120 125
   Ser Tyr Asp Arg Phe Leu Ser Val Thr Arg Ala Val Ser Tyr Arg Ala
   130 135 140
   Gln Gln Gly Asp Thr Arg Arg Ala Val Arg Lys Met Ala Leu Val Trp
   145 150 155 160
   Val Leu Ala Phe Leu Leu Tyr Gly Pro Ala Ile Leu Ser Trp Glu Tyr
   165 170 175
   Leu Ser Gly Gly Ser Ser Ile Pro Glu Gly His Cys Tyr Ala Glu Phe
   180 185 190
   Phe Tyr Asn Trp Tyr Phe Leu Ile Thr Ala Ser Thr Leu Glu Phe Phe
   195 200 205
   Thr Pro Phe Leu Ser Val Thr Phe Phe Asn Leu Ser Ile Tyr Leu Asn
   210 215 220
   Ile Gln Arg Arg Thr Arg Leu Arg Leu Asp Gly Gly Arg Glu Ala Gly
   225 230 235 240
   Pro Glu Pro Pro Pro Asp Ala Gln Pro Ser Pro Pro Pro Ala Pro Pro
   245 250 255
   Ser Cys Trp Gly Cys Trp Pro Lys Gly His Gly Glu Ala Met Pro Leu
   260 265 270
   His Ser Ser Gly Ser Ser Ser Arg Gly Thr Glu Arg Pro Arg Ser Leu
   275 280 285
   Lys Arg Gly Ser Lys Pro Ser Ala Ser Ser Ala Ser Leu Glu Lys Arg
   290 295 300
   Met Lys Met Val Ser Gln Ser Ile Thr Gln Arg Phe Arg Leu Ser Arg
   305 310 315 320
   Asp Lys Lys Val Ala Lys Ser Leu Ala Ile Ile Val Ser Ile Phe Gly
   325 330 335
   Leu Cys Trp Ala Pro Tyr Thr Leu Leu Met Ile Ile Arg Ala Ala Cys
   340 345 350
   His Gly Arg Cys Ile Pro Asp Tyr Trp Tyr Glu Thr Ser Phe Trp Leu
   355 360 365
   Leu Trp Ala Asn Ser Ala Val Asn Pro Val Leu Tyr Pro Leu Cys His
   370 375 380
   Tyr Ser Phe Arg Arg Ala Phe Thr Lys Leu Leu Cys Pro Gln Lys Leu
   385 390 395 400
   Lys Val Gln Pro His Gly Ser Leu Glu Gln Cys Trp Lys
   405 410
<210> 2
   <211> 1239
   <212> DNA
   <213> Rattus norvegicus
<220>
   <221> CDS
   <222> (1)..(1239)
<400> 2
   atg gag cgc gcg ccg ccc gac ggg ctg atg aac gcg tcg ggc act ctg 48
   Met Glu Arg Ala Pro Pro Asp Gly Leu Met Asn Ala Ser Gly Thr Leu
   1 5 10 15
   gcc gga gag gcg gcg gct gca ggc ggg gcg cgc ggc ttc tcg gct gcc 96
   Ala Gly Glu Ala Ala Ala Ala Gly Gly Ala Arg Gly Phe Ser Ala Ala
   20 25 30
   tgg acc gct gtc ctg gct gcg ctc atg gcg ctg ctc atc gtg gcc aca      144
   Trp Thr Ala Val Leu Ala Ala Leu Met Ala Leu Leu Ile Val Ala Thr
           35                  40                  45
   gta ctg ggc aac gcg ctg gtc atg ctc gcc ttc gtg gcg gat tcg agc      192
   Val Leu Gly Asn Ala Leu Val Met Leu Ala Phe Val Ala Asp Ser Ser
       50                  55                  60
   ctc cgc acc cag aac aac ttc ttt ctg ctc aac ctc gcc atc tcc gac      240
   Leu Arg Thr Gln Asn Asn Phe Phe Leu Leu Asn Leu Ala Ile Ser Asp
   65                  70                  75                  80
   ttc ctc gtg ggt gcc ttc tgc atc cca ttg tac gta ccc tat gtg ctg      288
   Phe Leu Val Gly Ala Phe Cys Ile Pro Leu Tyr Val Pro Tyr Val Leu
                   85                  90                  95
   acc ggc cgt tgg acc ttc ggc cgg ggc ctc tgc aag ctg tgg ctg gtg      336
   Thr Gly Arg Trp Thr Phe Gly Arg Gly Leu Cys Lys Leu Trp Leu Val
               100                 105                 110
   gta gac tac cta ctg tgt gcc tcc tcg gtc ttc aac atc gta ctc atc      384
   Val Asp Tyr Leu Leu Cys Ala Ser Ser Val Phe Asn Ile Val Leu Ile
           115                 120                 125
   agc tat gac cga ttc ctg tca gtc act cga gct gtc tcc tac agg gcc      432
   Ser Tyr Asp Arg Phe Leu Ser Val Thr Arg Ala Val Ser Tyr Arg Ala
       130                 135                 140
   cag cag ggg gac acg aga cgg gcc gtt cgg aag atg gca ctg gtg tgg      480
   Gln Gln Gly Asp Thr Arg Arg Ala Val Arg Lys Met Ala Leu Val Trp
   145                 150                 155                 160
   gtg ctg gcc ttc ctg ctg tat ggg cct gcc atc ctg agt tgg gag tac      528
   Val Leu Ala Phe Leu Leu Tyr Gly Pro Ala Ile Leu Ser Trp Glu Tyr
                   165                 170                 175
   ctg tct ggt ggc agt tcc atc ccc gag ggc cac tgc tat gct gag ttc      576
   Leu Ser Gly Gly Ser Ser Ile Pro Glu Gly His Cys Tyr Ala Glu Phe
               180                 185                 190
   ttc tac aac tgg tac ttt ctc atc acg gcc tcc acc ctc gag ttc ttc      624
   Phe Tyr Asn Trp Tyr Phe Leu Ile Thr Ala Ser Thr Leu Glu Phe Phe
           195                 200                 205
   acg ccc ttc ctc agc gtt acc ttc ttc aac ctc agc atc tac ctg aac      672
   Thr Pro Phe Leu Ser Val Thr Phe Phe Asn Leu Ser Ile Tyr Leu Asn
       210                 215                 220
   atc cag agg cgc acc cgc ctt cgg ctt gat ggg ggc cgt gag gct ggc      720
   Ile Gln Arg Arg Thr Arg Leu Arg Leu Asp Gly Gly Arg Glu Ala Gly
   225                 230                 235                 240
   cca gaa ccc cca cca gat gcc cag ccc tcg cca cct cca gct ccc ccc      768
   Pro Glu Pro Pro Pro Asp Ala Gln Pro Ser Pro Pro Pro Ala Pro Pro
                   245                 250                 255
   agc tgc tgg ggc tgc tgg cca aaa ggg cat ggc gag gcc atg ccg ttg      816
   Ser Cys Trp Gly Cys Trp Pro Lys Gly His Gly Glu Ala Met Pro Leu
               260                 265                 270
   cac agc tct ggc agc tcc tca agg ggc act gag agg cca cgc tca ctc      864
   His Ser Ser Gly Ser Ser Ser Arg Gly Thr Glu Arg Pro Arg Ser Leu
           275                 280                 285
   aaa agg ggc tcc aag cca tca gca tct tca gca tcc ctg gag aag cgc      912
   Lys Arg Gly Ser Lys Pro Ser Ala Ser Ser Ala Ser Leu Glu Lys Arg
       290                 295                 300
   atg aag atg gtg tcc cag agc atc acc cag cgc ttc cgg ctg tcg cgg      960
   Met Lys Met Val Ser Gln Ser Ile Thr Gln Arg Phe Arg Leu Ser Arg
   305                 310                 315                 320
   gac aag aag gtg gcc aag tcg ctg gcc atc atc gtg agc atc ttt ggg     1008
   Asp Lys Lys Val Ala Lys Ser Leu Ala Ile Ile Val Ser Ile Phe Gly
                   325                 330                 335
   ctc tgc tgg gcg ccg tac acg ctc cta atg atc atc cga gct gct tgc     1056
   Leu Cys Trp Ala Pro Tyr Thr Leu Leu Met Ile Ile Arg Ala Ala Cys
               340                 345                 350
   cat ggc cgc tgc atc ccc gat tac tgg tac gag acg tcc ttc tgg ctt     1104
   His Gly Arg Cys Ile Pro Asp Tyr Trp Tyr Glu Thr Ser Phe Trp Leu
               355                 360                 365
   ctg tgg gcc aac tcg gcc gtc aac ccc gtc ctc tac cca ctg tgc cac      1152
   Leu Trp Ala Asn Ser Ala Val Asn Pro Val Leu Tyr Pro Leu Cys His
       370                 375                 380
   tac agc ttc cgc aga gcc ttc acc aag ctc ctc tgc ccc cag aag ctc      1200
   Tyr Ser Phe Arg Arg Ala Phe Thr Lys Leu Leu Cys Pro Gln Lys Leu
   385                 390                 395                 400
   aag gtc cag ccc cac ggc tcc ctg gag cag tgc tgg aag                  1239
   Lys Val Gln Pro His Gly Ser Leu Glu Gln Cys Trp Lys
                   405                 410
<210> 3
   <211> 21
   <212> DNA
   <213> Artificial Sequence
<220>
   <223> Description of Artificial Sequence: Artificially synthesized primer sequence
<400> 3
   batngccaac ctbkccttct c 21
<210> 4
   <211> 20
   <212> DNA
   <213> Artificial Sequence
<220>
   <223> Description of Artificial Sequence: Artificially synthesized primer sequence
<400> 4
   ccataaaagn nggggttgac 20
<210> 5
   <211> 2700
   <212> DNA
   <213> Rattus norvegicus
<220>
   <221> CDS
   <222> (351)..(1589)
<400> 5
<210> 6
   <211> 29
   <212> DNA
   <213> Artificial Sequence
<220>
   <223> Description of Artificial Sequence: Artificially synthesized primer sequence
<400> 6
   cgaggatccg tgaggctccg gtgcccgtc 29
<210> 7
   <211> 32
   <212> DNA
   <213> Artificial Sequence
<220>
   <223> Description of Artificial Sequence: Artificially synthesized primer sequence
<400> 7
   cgggtaagct tcacgacacc tgaaatggaa ga 32
<210> 8
   <211> 24
   <212> DNA
   <213> Artificial Sequence
<220>
   <223> Description of Artificial Sequence: Artificially synthesized primer sequence
<400> 8
   ccttctgcat cccattgtac gtacc 24
<210> 9
   <211> 21
   <212> DNA
   <213> Artificial Sequence
<220>
   <223> Description of Artificial Sequence: Artificially synthesized primer sequence
<400> 9
   cttccgccgg gccttcacca a 21
<210> 10
   <211> 24
   <212> DNA
   <213> Artificial Sequence
<220>
   <223> Description of Artificial Sequence: Artificially synthesized primer sequence
<400> 10
   acagacacgg cggggctcac 20
<210> 11
   <211> 1350
   <212> DNA
   <213> Homo sapiens
<220>
   <221> exon
   <222> (280)..(557)
<400> 11
   gcactcggct gcgcgttgcn tccggctgca cggtcgcacc ggcagcggct caggctccgg 60
   ctcctctccc gctgcagcag ccgcgctgcc ggccccactg ggctcggatc cggccccggc 120
   cccctcggca ccgcctgctc tggccccggc cccggccccg cggaccatgc gctgggcgcc 180
   cccaggggaa cccgacccgg ccaagggccc gcaaagacga ggctcccggg ccggggcccc 240
   tcccggccgc ccagctctcg gccggcgccc tgccccgcgt cccggagccg cgtgagcctg 300
   cggggccatg gagcgcgcgc cgcccgacgg gccgctgaac gcttcggggg cgctggcggg 360
   cgaggcggcg gcggcgggcg gggcgcgcgg cttctcggca gcctggaccg cggtgctggc 420
   cgcgctcatg gcgctgctca tcgtggccac ggtgctgggc aacgcgctgg tcatgctcgc 480
   cttcgtggcc gactcgagcc tccgcaccca gaacaacttc ttcctgctca acctcgccat 540
   ctccgacttc ctcgtcggta aatccccagc ccctggccgc tggggaccca ggggcgccca 600
   gcgtggccgg gccagcgggg actggaacac ggacctgggt ggctcccgca ggcacacgcc 660
   ccaccagggg acccggcctg ggaagggggc gtccggagcc catggggtgg ggggcacagg 720
   cgaagttcct tgccactcag gcctcgggac aggggctggg gagagatgtc cccgggaagg 780
   gacacgggca ctgggcgagg cgcaaggcgc aaaggcagcg ggtgcagctc tggctcctgc 840
   gctgtagcca aacaaaggct gctgcggact taggacgcgc ggagggcgca gtggggcggt 900
   ttagagaagg tctgggggag gggacatgga agggggattt ttagagctgt gttgggggaa 960
   gggacggtgg ggaaggtggg ggttggggga gacgctcgga ggagcgtgct ctcacgtgtc 1020
   caggctctgc tgccggctgg ggggcggggc acgcggaggg ggctggagcg ccagacacct 1080
   gttggggctg tgaggtgcgt ctcccagacg ctccaagccc gcttggcagt agtagtagcg 1140
   gctggcggct ggcggctgca accaagtgcc ctttcagcca ggagaaaggc tttctccttg 1200
   tctaagctga gaccgagggt tgtccagcgc cagggtaggg gctggagtcc agcgggggag 1260
   gggagaagga aattgtcttc tttcctcctt tgagggctgg gagggctgga cagaagtcca 1320
   gggaatcccg actccaggct ctcgggggtc 1350
<210> 12
   <211> 448
   <212> DNA
   <213> Homo sapiens
<220>
   <221> exon
   <222> (259)..(425)
<400> 12
   gagctcccca tgcctggatc atccctcctg cccccaggcc caggggacac agatagtgct 60
   gggagctatg tgggggtgaa ggctggcggc agggcagagt ttgtggctga caccaggtgg 120
   aggggtggta agatgaggat ggctagttcc agaaaagcag ccaccatgtg accccaggtc 180
   ccgccggtgt ctgcgcttag gtccgtctgt cccctggccc ctggctgcat ggtcccactg 240
   tggccctact ccccacaggc gccttctgca tcccactgta tgtaccctac gtgctgacag 300
   gccgctggac cttcggccgg ggcctctgca agctgtggct ggtagtggac tacctgctgt 360
   gcacctcctc tgccttcaac atcgtgctca tcagctacga ccgcttcctg tcggtcaccc 420
   gagcggtgag tcctgggctg cggagctc 448
<210> 13
   <211> 1893
   <212> DNA
   <213> Homo sapiens
<220>
   <221> exon
   <222> (293)..(1209)
<400> 13
   gagctcacag ctggtagggg gtggtaaaca ggcagcctag cagagagtga gggttcaggt 60
   tggtcccagg gagcttctga ggctctcact gagtgtggca gggcaccagt ccgggacccc 120
   agtggggagg gttagaggaa gggaggggaa agagggaggg agggaggaca ggaggggaaa 180
   ggaggagcat tgctgctgag ggaagggccc acataggggc ccacaggcta cgggggcgca 240
   cccagcccaa tattccttcc gccccgcccc tgaccagcct gcccttctgc aggtctcata 300
   ccgggcccag cagggtgaca cgcggcgggc agtgcggaag atgctgctgg tgtgggtgct 360
   ggccttcctg ctgtacggac cagccatcct gagctgggag tacctgtccg ggggcagctc 420
   catccccgag ggccactgct atgccgagtt cttctacaac tggtacttcc tcatcacggc 480
   ttccaccctg gagttcttta cgcccttcct cagcgtcacc ttctttaacc tcagcatcta 540
   cctgaacatc cagaggcgca cccgcctccg gctggatggg gctcgagagg cagccggccc 600
   cgagccccct cccgaggccc agccctcacc acccccaccg cctggctgct ggggctgctg 660
   gcagaagggg cacggggagg ccatgccgct gcacaggtat ggggtgggtg aggcggccgt 720
   aggcgctgag gccggggagg cgaccctcgg gggtggcggt gggggcggct ccgtggcttc 780
   acccacctcc agctccggca gctcctcgag gggcactgag aggccgcgct cactcaagag 840
   gggctccaag ccatcggcgt cctcggcctc actggagaag cgcatgaaga tggtgtccca 900
   gagcttcacc cagcgctttc ggctgtctcg ggacaggaaa gtggccaagt cgctggccgt 960
   catcgtgagc atctttgggc tctgctgggc cccatacacg ctgctgatga tcatccgggc 1020
   cgcctgccat ggccactgcg tccctgacta ctggtacgaa acctccttct ggctcctgtg 1080
   ggccaactcg gctgtcaacc ctgtcctcta ccctctgtgc caccacagct tccgccgggc 1140
   cttcaccaag ctgctctgcc cccagaagct caaaatccag ccccacagct ccctggagca 1200
   ctgctggaag tgagtggccc accagagcct ccctcagcca cgcctctctc agcccaggtc 1260
   tcctgggcat ctggccctgc tgccccctac ccggctcgtt cccccagggg tgagccccgc 1320
   cgtgtctgtg gccctctctt aatgccacgg cagccaccct gccatggagg cgccttcctg 1380
   ggttggccag agggcccctc actggctgga ctggaggctg ggtggccggc cctgcccccc 1440
   acattctggc tccaccggga gggacagtct ggaggtccca gacatgctgc ccaccccctg 1500
   ctggtgccca cccttcgcag ttactggttg gtgttcttcc caaagcaagc acctgggtgt 1560
   gctccaggct tcctgcccta gcagtttgcc tctgcacgtg cacacacctg cacacccctg 1620
   cacacacctg cacaccgtcc ctctccccgg acaagcccag gacactgcct ttgctgcctt 1680
   ctgtctcttg cataagcctc aggcctggcc ctttcacccc tcttcccacc aactctctct 1740
   gcccccaaaa gtgtcaaggg gccctaggaa cctcgaagct gttctctgct tttccattct 1800
   gggtgttttc agaaagatga agaagaaaac atgtctgtga acttgatgtt cctgggatgt 1860
   ttaatcaaga gagacaaaat tgctgaggag ctc 1893
<210> 14
   <211> 20
   <212> DNA
   <213> Artificial Sequence
<220>
   <223> Description of Artificial Sequence: Artificially synthesized primer sequence
<400> 14
   tgaacgcttc gggggcgctg 20
<210> 15
   <211> 21
   <212> DNA
   <213> Artificial Sequence
<220>
   <223> Description of Artificial Sequence: Artificially synthesized primer sequence
<400> 15
   gagatggcga ggttgagcag g 21
<210> 16
   <211> 20
   <212> DNA
   <213> Artificial Sequence
<220>
   <223> Description of Artificial Sequence: Artificially synthesized primer sequence
<400> 16
   ggctccaagc catcggcgtc 20
<210> 17
   <211> 20
   <212> DNA
   <213> Artificial Sequence
<220>
   <223> Description of Artificial Sequence: Artificially synthesized primer sequence
<400> 17
   ctcacttcca gcagtgctcc 20
<210> 18
   <211> 20
   <212> DNA
   <213> Artificial Sequence
<220>
   <223> Description of Artificial Sequence: Artificially synthesized primer sequence
<400> 18
   gcctccgcac ccagaacaac 20
<210> 19
   <211> 19
   <212> DNA
   <213> Artificial Sequence
<220>
   <223> Description of Artificial Sequence: Artificially synthesized primer sequence
<400> 19
   tgcgcctctg gatgttcag 19
<210> 20
   <211> 453
   <212> PRT
   <213> Homo sapiens
<400> 20
   Met Glu Arg Ala Pro Pro Asp Gly Pro Leu Asn Ala Ser Gly Ala Leu
   1 5 10 15
   Ala Gly Glu Ala Ala Ala Ala Gly Gly Ala Arg Gly Phe Ser Ala Ala
   20 25 30
   Trp Thr Ala Val Leu Ala Ala Leu Met Ala Leu Leu Ile Val Ala Thr
   35 40 45
   Val Leu Gly Asn Ala Leu Val Met Leu Ala Phe Val Ala Asp Ser Ser
   50 55 60
   Leu Arg Thr Gln Asn Asn Phe Phe Leu Leu Asn Leu Ala Ile Ser Asp
   65 70 75 80
   Phe Leu Val Gly Ala Phe Cys Ile Pro Leu Tyr Val Pro Tyr Val Leu
   85 90 95
   Thr Gly Arg Trp Thr Phe Gly Arg Gly Leu Cys Lys Leu Trp Leu Val
   100 105 110
   Val Asp Tyr Leu Leu Cys Thr Ser Ser Ala Phe Asn Ile Val Leu Ile
   115 120 125
   Ser Tyr Asp Arg Phe Leu Ser Val Thr Arg Ala Val Ser Tyr Arg Ala
   130 135 140
   Gln Gln Gly Asp Thr Arg Arg Ala Val Arg Lys Met Leu Leu Val Trp
   145 150 155 160
   Val Leu Ala Phe Leu Leu Tyr Gly Pro Ala Ile Leu Ser Trp Glu Tyr
   165 170 175
   Leu Ser Gly Gly Ser Ser Ile Pro Glu Gly His Cys Tyr Ala Glu Phe
   180 185 190
   Phe Tyr Asn Trp Tyr Phe Leu Ile Thr Ala Ser Thr Leu Glu Phe Phe
   195 200 205
   Thr Pro Phe Leu Ser Val Thr Phe Phe Asn Leu Ser Ile Tyr Leu Asn
   210 215 220
   Ile Gln Arg Arg Thr Arg Leu Arg Leu Asp Gly Ala Arg Glu Ala Ala
   225 230 235 240
   Gly Pro Glu Pro Pro Pro Glu Ala Gln Pro Ser Pro Pro Pro Pro Pro
   245 250 255
   Gly Cys Trp Gly Cys Trp Gln Lys Gly His Gly Glu Ala Met Pro Leu
   260 265 270
   His Arg Tyr Gly Val Gly Glu Ala Ala Val Gly Ala Glu Ala Gly Glu
   275 280 285
   Ala Thr Leu Gly Gly Gly Gly Gly Gly Gly Ser Val Ala Ser Pro Thr
   290 295 300
   Ser Ser Ser Gly Ser Ser Ser Arg Gly Thr Glu Arg Pro Arg Ser Leu
   305 310 315 320
   Lys Arg Gly Ser Lys Pro Ser Ala Ser Ser Ala Ser Leu Glu Lys Arg
   325 330 335
   Met Lys Met Val Ser Gln Ser Phe Thr Gln Arg Phe Arg Leu Ser Arg
   340 345 350
   Asp Arg Lys Val Ala Lys Ser Leu Ala Val Ile Val Ser Ile Phe Gly
   355 360 365
   Leu Cys Trp Ala Pro Tyr Thr Leu Leu Met Ile Ile Arg Ala Ala Cys
   370 375 380
   His Gly His Cys Val Pro Asp Tyr Trp Tyr Glu Thr Ser Phe Trp Leu
   385 390 395 400
   Leu Trp Ala Asn Ser Ala Val Asn Pro Val Leu Tyr Pro Leu Cys His
   405 410 415
   His Ser Phe Arg Arg Ala Phe Thr Lys Leu Leu Cys Pro Gln Lys Leu
   420 425 430
   Lys Ile Gln Pro His Ser Ser Leu Glu His Cys Trp Lys Lys Met Lys
   435 440 445
   Lys Lys Thr Cys Leu
   450
<210> 21
   <211> 2050
   <212> DNA
   <213> Homo sapiens
<220>
   <221> CDS
   <222> (271)..(1629)
<400> 21
   agagatgtag ggcgcccctt ttagctgcgc acagaacgaa agaactcgtt ttttctttaa 60
   gtgagtgtgc ttgggtgacg cttagggcgc cctccgcagt gcgcgcagga aagcgcactg 120
   aggctgcgga ggcagagctg catgctgggt gcgggaagag gtgggctccg tcgcggagtc 180
   gctgagtccg tgccctttta gttagttctg cagtctagta tggtccccat ttgcccttcc 240
   actcccggag ccgcgtgagc ctgcggggcc atg gag cgc gcg ccg ccc gac ggg 294
   Met Glu Arg Ala Pro Pro Asp Gly
   1 5
   ccg ctg aac gct tcg ggg gcg ctg gcg ggc gag gcg gcg gcg gcg ggc 342
   Pro Leu Asn Ala Ser Gly Ala Leu Ala Gly Glu Ala Ala Ala Ala Gly
   10 15 20
   ggg gcg cgc ggc ttc tcg gca gcc tgg acc gcg gtg ctg gcc gcg ctc   390
   Gly Ala Arg Gly Phe Ser Ala Ala Trp Thr Ala Val Leu Ala Ala Leu
    25                  30                  35                  40
   atg gcg ctg ctc atc gtg gcc acg gtg ctg ggc aac gcg ctg gtc atg   438
   Met Ala Leu Leu Ile Val Ala Thr Val Leu Gly Asn Ala Leu Val Met
                    45                  50                  55
   ctc gcc ttc gtg gcc gac tcg agc ctc cgc acc cag aac aac ttc ttc   486
   Leu Ala Phe Val Ala Asp Ser Ser Leu Arg Thr Gln Asn Asn Phe Phe
                60                  65                  70
   ctg ctc aac ctc gcc atc tcc gac ttc ctc gtc ggc gcc ttc tgc atc   534
   Leu Leu Asn Leu Ala Ile Ser Asp Phe Leu Val Gly Ala Phe Cys Ile
            75                  80                  85
   cca ctg tat gta ccc tac gtg ctg aca ggc cgc tgg acc ttc ggc cgg   582
   Pro Leu Tyr Val Pro Tyr Val Leu Thr Gly Arg Trp Thr Phe Gly Arg
        90                  95                 100
   ggc ctc tgc aag ctg tgg ctg gta gtg gac tac ctg ctg tgc acc tcc   630
   Gly Leu Cys Lys Leu Trp Leu Val Val Asp Tyr Leu Leu Cys Thr Ser
   105                 110                 115                 120
   tct gcc ttc aac atc gtg ctc atc agc tac gac cgc ttc ctg tcg gtc   678
   Ser Ala Phe Asn Ile Val Leu Ile Ser Tyr Asp Arg Phe Leu Ser Val
                   125                 130                 135
   acc cga gcg gtc tca tac cgg gcc cag cag ggt gac acg cgg cgg gca   726
   Thr Arg Ala Val Ser Tyr Arg Ala Gln Gln Gly Asp Thr Arg Arg Ala
               140                 145                 150
   gtg cgg aag atg ctg ctg gtg tgg gtg ctg gcc ttc ctg ctg tac gga   774
   Val Arg Lys Met Leu Leu Val Trp Val Leu Ala Phe Leu Leu Tyr Gly
           155                 160                 165
   cca gcc atc ctg agc tgg gag tac ctg tcc ggg ggc agc tcc atc ccc   822
   Pro Ala Ile Leu Ser Trp Glu Tyr Leu Ser Gly Gly Ser Ser Ile Pro
       170                 175                 180
   gag ggc cac tgc tat gcc gag ttc ttc tac aac tgg tac ttc ctc atc   870
   Glu Gly His Cys Tyr Ala Glu Phe Phe Tyr Asn Trp Tyr Phe Leu Ile
   185 190 195 200
   acg gct tcc acc ctg gag ttc ttt acg ccc ttc ctc agc gtc acc ttc 918
   Thr Ala Ser Thr Leu Glu Phe Phe Thr Pro Phe Leu Ser Val Thr Phe
   205 210 215
   ttt aac ctc agc atc tac ctg aac atc cag agg cgc acc cgc ctc cgg 966
   Phe Asn Leu Ser Ile Tyr Leu Asn Ile Gln Arg Arg Thr Arg Leu Arg
   220 225 230
   ctg gat ggg gct cga gag gca gcc ggc ccc gag ccc cct ccc gag gcc 1014
   Leu Asp Gly Ala Arg Glu Ala Ala Gly Pro Glu Pro Pro Pro Glu Ala
   235 240 245
   cag ccc tca cca ccc cca ccg cct ggc tgc tgg ggc tgc tgg cag aag 1062
   Gln Pro Ser Pro Pro Pro Pro Pro Gly Cys Trp Gly Cys Trp Gln Lys
   250 255 260
   ggg cac ggg gag gcc atg ccg ctg cac agg tat ggg gtg ggt gag gcg 1110
   Gly His Gly Glu Ala Met Pro Leu His Arg Tyr Gly Val Gly Glu Ala
   265 270 275 280
   gcc gta ggc gct gag gcc ggg gag gcg acc ctc ggg ggt ggc ggt ggg 1158
   Ala Val Gly Ala Glu Ala Gly Glu Ala Thr Leu Gly Gly Gly Gly Gly
   285 290 295
   ggc ggc tcc gtg gct tca ccc acc tcc agc tcc ggc agc tcc tcg agg 1206
   Gly Gly Ser Val Ala Ser Pro Thr Ser Ser Ser Gly Ser Ser Ser Arg
   300 305 310
   ggc act gag agg ccg cgc tca ctc aag agg ggc tcc aag ccg tcg gcg 1254
   Gly Thr Glu Arg Pro Arg Ser Leu Lys Arg Gly Ser Lys Pro Ser Ala
   315 320 325
   tcc tcg gcc tcg ctg gag aag cgc atg aag atg gtg tcc cag agc ttc 1302
   Ser Ser Ala Ser Leu Glu Lys Arg Met Lys Met Val Ser Gln Ser Phe
   330 335 340
   acc cag cgc ttt cgg ctg tct cgg gac agg aaa gtg gcc aag tcg ctg 1350
   Thr Gln Arg Phe Arg Leu Ser Arg Asp Arg Lys Val Ala Lys Ser Leu
   345 350 355 360
   gcc gtc atc gtg agc atc ttt ggg ctc tgc tgg gcc cca tac acg ctg 1398
   Ala Val Ile Val Ser Ile Phe Gly Leu Cys Trp Ala Pro Tyr Thr Leu
   365 370 375
   ctg atg atc atc cgg gcc gcc tgc cat ggc cac tgc gtc cct gac tac 1446
   Leu Met Ile Ile Arg Ala Ala Cys His Gly His Cys Val Pro Asp Tyr
   380 385 390
   tgg tac gaa acc tcc ttc tgg ctc ctg tgg gcc aac tcg gct gtc aac 1494
   Trp Tyr Glu Thr Ser Phe Trp Leu Leu Trp Ala Asn Ser Ala Val Asn
   395 400 405
   cct gtc ctc tac cct ctg tgc cac cac agc ttc cgc cgg gcc ttc acc 1542
   Pro Val Leu Tyr Pro Leu Cys His His Ser Phe Arg Arg Ala Phe Thr
   410 415 420
   aag ctg ctc tgc ccc cag aag ctc aaa atc cag ccc cac agc tcc ctg 1590
   Lys Leu Leu Cys Pro Gln Lys Leu Lys Ile Gln Pro His Ser Ser Leu
   425 430 435 440
   gag cac tgc tgg aaa aag atg aag aag aaa aca tgt ctg tgaacttgat 1639
   Glu His Cys Trp Lys Lys Met Lys Lys Lys Thr Cys Leu
   445 450
   gttcctggga tgtttaatca agagagacaa aattgctgag gagctcaggg ctggattggc 1699
   aggtgtgggc tcccacgccc tcctccctcc gctaaggctt ccggctgagc tgtgccagct 1759
   gcttctgccc accccgcctc tgggctcaca ccagccctgg tggccaagcc tgccccggcc 1819
   actctgtttg ctcacccagg acctctgggg gttgttggga ggagggggcc cggctgggcc 1879
   cgagggtccc aaggcgtgca ggggcggtcc agaggaggtg cccgggcagg ggccgcttcg 1939
   ccatgtgctg tgcacccgtg ccacgcgctc tgcatgctcc tctgcctgtg cccgctgcgc 1999
   tgccctgcaa accgtgaggt cacaataaag tgtatttttt tattggtgct g 2050
<210> 22
   <211> 20
   <212> DNA
   <213> Artificial Sequence
<220>
   <223> Description of Artificial Sequence: Artificially synthesized primer sequence
<400> 22
   tgcatcccat tgtacgtncc 20
<210> 23
   <211> 20
   <212> DNA
   <213> Artificial Sequence
<220>
   <223> Description of Artificial Sequence: Artificially synthesized primer sequence
<400> 23
   atcattagga gcgtgtangg 20
<210> 24
   <211> 20
   <212> DNA
   <213> Artificial Sequence
<220>
   <223> Description of Artificial Sequence: Artificially synthesized primer sequence
<400> 24
   tgctctggga caccatcttc 20

## Description

### Technical Field

The present invention relates to a novel guanosine triphosphate binding protein-coupled receptor protein, a DNA encoding said protein, and a method for screening drug-candidate compounds using them.

### Background Art

Many hormones and neurotransmitters regulate physiological functions through specific receptor proteins located on the cell membrane. Many of these receptor-proteins transduce signals into the cell by activating a guanosine triphosphate binding protein (occasionally referred to as "G protein" below) that is coupled to them. These receptor proteins are thereby named as G protein-coupled receptors. Since they have a common structure composed of seven transmembrane regions, they are also generally called "seven-transmembrane receptor proteins."

G protein-coupled receptors, which are expressed on the surface of cells *in vivo* and functioning cells of tissues, play an extremely important role as a target of molecules such as hormones, neurotransmitters, and biologically active compounds, which regulate the functions of these cells and tissues. Therefore, G protein-coupled receptor proteins have received great attention as targets in drug-development.

G protein-coupled receptors reported so far include, muscarinic acetylcholine receptors M1, M2, M3, and M4 (Peralta E.G. et al., EMBO J. 6:3923-3929 (1987)), muscarinic acetylcholine receptor M5 (Bonner T. I. et al., Neuron 1:403-410 (1988)), adenosine receptor A1 (Libert F. et al., Science 244:569-572 (1989)), α1A adrenoreceptor (Bruno J.F. et al., Biochem. Biophys. Res. Commun. 179:1485-1490 (1991)), β1 adrenoreceptor (Frielle T. et al., Proc. Natl. Acad. Sci. USA 84:7920-7924 (1987)), angiotensin receptor AT₁ (Takayanagi R. et al., Biochem. Biophys. Res. Commun. 183:910-916 (1992)), endothelin receptor ET_{A} (Adachi M. et al., Biochem. Biophys. Res. Commun. 180:1265-1272 (1991)), gonadotropin releasing factor receptor (Kaker S.S. et al., Biochem. Biophys. Res. Commun. 189:289-295 (1992)), histamine receptor H₂ (Ruat M. et al., Proc. Natl. Acad. Sci. USA 87:1658-1672 (1992)), neuropeptide Y receptor Y1 (Larhammar D. et al., J. Biol. Chem. 267:10935-10938 (1992)), interleukin-8 receptor IL8R_{A} (Holmes W.E. et al., Science 2563:1278-1280(1991)), dopamine receptor D₁ (Mahan L.C. et al., Proc. Natl. Acad. Sci. USA 87:2196-2200 (1990)), metabolic glutamate receptor mGluR1 (Masu M. et al., Nature 349:760-765 (1991)), and somatostatin receptor SS₁ (Yamada Y. et al., Proc. Natl. Acad. Sci. USA 89:251-255) (for reference, Watson S. and Arkinstall S., The G protein Linked Receptor FactsBook, Academic Press (1994)). Examples of developed medicines aimed at G protein-coupled receptors are: terazosine hydrochloride (antihypertensive agent, α1 adrenoreceptor antagonist), atenolol (antiarrhythmia, β1 adrenoreceptor antagonist), dicyclomine hydrochloride (antispasmodic agent, acetylcholine receptor antagonist), ranitidine hydrochloride (drug for peptic ulcers, histamine receptor H2 antagonist), trazodone hydrochloride (antidepressant, serotonin receptor 5-HT1B antagonist), and buprenorphine hydrochloride (analgesic agent, opioid receptor κ agonist) (for reference, Stadel J.M. et al., Trends Pharm. Sci. 18:430-437 (1997); Medicine Handbook 5th edition, Yakugyo-Jiho).

The hypothalamus, a part of the brain which governs a number of programs that trigger a particular response, contributes to the homeostasis of the internal environment by means of a variety of outputs, as the center of the autonomic nervous system. For instance, it releases hormones such as thyrotropic hormone releasing hormone, gonadotropic hormone releasing hormone, and growth hormone releasing hormone, and thereby regulates the entire endocrine system through the actions of these hormones on the specific receptors expressed in target cells. These outputs in the hypothalamus are thought to be mediated by receptors expressed in the hypothalamus and compounds reacting with them. Therefore, elucidation of the relationship between the compounds regulating the hypothalamus outputs and their specific receptors expressed in the hypothalamus is extremely important in developing novel medicines for the treatment of diseases arising from endocrine disorders.

### Disclosure of the Invention

The present invention provides a novel G protein-coupled receptor protein expressed in the brain (in particular, thalamus and hypothalamus, etc.). It also provides a method for screening ligands and drug-candidate compounds using said receptor protein.

The inventors first selected a region highly conserved in known G protein-coupled receptor proteins, then designed primers corresponding to the region, and performed reverse transcriptase-polymerase chain reaction (RT-PCR) using mRNA obtained from rat thalamus and hypothalamus. Next, amplified clones were randomly selected, and their partial nucleotide sequences were determined. To remove known clones from the nucleotide sequence determined-clones, colony-hybridization was performed using as a probe, cDNA clones judged to be encoding a known G protein-coupled receptor protein by homology search. Negative clones that failed to hybridize with any probe were selected. Using probes prepared based on the nucleotide sequence of the negative clones, the inventors screened cDNA libraries from rat thalamus and hypothalamus, and succeeded in isolating a full-length cDNA encoding a rat G protein-coupled receptor. They also succeeded in isolating a full-length human cDNA corresponding to the rat cDNA. Furthermore, northern blot analysis of the tissue specificity of the gene expression showed that these genes are specifically expressed in the brain.

These G protein-coupled receptors would be extremely useful in screening for ligands and compounds that regulate the signal transduction from receptors, which are anticipated to be utilized as novel medicines.

Thus, the present invention relates to novel G protein-coupled receptor proteins that are expressed in the brain, DNAs encoding them, and a method for screening ligands and compounds that are drug-candidate compounds using the proteins. Specifically, the invention relates to:
(1) a DNA encoding a guanosine triphosphate binding protein-coupled receptor protein selected from the group consisting of
   (a) a DNA comprising the nucleotide sequence of SEQ ID NO:2 or 21;
   (b) a DNA encoding a polypeptide comprising the amino acid sequence of SEQ ID NO: 1, or 20;
   (c) a DNA with a sequence identity of not less than 90% to the entire nucleotide sequence of the DNA of SEQ ID NO:2 or 21 or a DNA as defined in (a); and
   (d) a DNA encoding a polypeptide having the amino acid sequence of (a) or (b), wherein one to three amino acids are replaced, deleted or added;
(2) a vector comprising the DNA of (1);
(3) a transformant carrying the vector of (2);
(4) a method of producing a guanosine triphosphate binding protein-coupled receptor protein comprising culturing the transformant of (3);
(5) a polypeptide encoded by the DNA of (1) or obtainable by the process of (4);
(6) an antibody which binds to the polypeptide of (5);
(7) a method of screening for a ligand of the polypeptide of (5); the method comprising exposing a test compound to the polypeptide of (5) and selecting a compound that binds to said polypeptide;
(8) a method of screening for a compound that inhibits the binding between the polypeptide of (5) and its ligand, the method comprising
   (a) exposing a ligand to the polypeptide of claim 5 in the presence of a test compound and detecting the binding activity between the polypeptide of (5) and the ligand; and
   (b) comparing the binding activity detected in (a) with that in the absence of the test compound, and selecting a compound that reduces the binding activity between the polypeptide of (5) and the ligand;
(9) a kit for screening a compound that inhibits the binding between the polypeptide of (5) and the ligand, the kit comprising the polypeptide of (5);
(10) a pharmaceutical composition comprising the DNA of (1), the polypeptide of (5) or the antibody of (6);
(11) use of the DNA of (1) or the antibody of (6) for the preparation of a pharmaceutical composition for the treatment of diseases arising from the abnormal expression of a guanosine triphosphate binding protein-coupled receptors or diseases that arise from a mutation in said receptor;
(12) use of the polypeptide of (5) for the preparation of a pharmaceutical composition for the treatment of disabilities in memory and learning, diseases relating to the regulation of blood pressure, digestion, body temperature or food intake or other diseases relating to the control of the autonomous nervous system.

"G protein-coupled receptor protein" herein refers to a receptor protein that transduces intracellular signals by activating G proteins. "Ligand" refers to a natural compound capable of binding to a G protein-coupled receptor and inducing signal transduction. "Agonist" refers to a compound having a bioactivity similar to that of the ligands of G protein-coupled receptors, including both natural and artificially synthesized compounds. "Antagonist" refers to a compound capable of inhibiting the bioactivity of a ligand of a G protein-coupled receptor, including both natural and artificially synthesized compounds. "Protein" and "peptide" as used herein include their salts as well.

The present invention relates to a novel G protein-coupled receptor protein. The nucleotide sequence of the cDNA encoding rat G protein-coupled receptor "BG2" isolated in the present invention is shown as SEQ ID NO: 2, and the amino acid sequence of the "BG2" protein is shown as SEQ ID NO: 1. The nucleotide sequence of the cDNA encoding human G protein-coupled receptor "BG2" isolated herein is shown as SEQ ID NO: 21, and the amino acid sequence of the human "BG2" protein is shown as SEQ ID NO: 20.

Rat "BG2" protein has 26%, 25%, and 29% homology to known G protein-coupled receptors; bovine muscarinic acetylcholine receptor M3 protein (Lee P.H. et al., Biochim. Biophys. Acta 1223:151-154 (1994)), human muscarinic acetylcholine receptor M5 protein (Bonner T.I. et al., Neuron 1:403-410 (1988)), and mouse α2A adrenoreceptor (Link R. et al., Mol. Pharmacol. 42:16-27 (1992)), respectively. The result of the hydrophobicity plot analysis showed that rat "BG2" protein contains hydrophobic regions (seven transmembrane regions) characteristic to G protein-coupled receptors. In addition, the coding region of the rat "BG2" cDNA has a size of approximately 1.2 kb, a size similar to that of the known G protein-coupled receptors.

Human "BG2" protein has 32%, 28%, and 27% homology to known G protein-coupled receptors; human α-2C-1 adrenoreceptor (Regan J.W. et al., Proc. Natl. Acad. Sci. USA 85:6301-6305 (1988)), mouse β-1 adrenoreceptor (Jasper J.R. et al., Biochim. Biophys. Acta 1178:307-309 (1993)), and human muscarinic acetylcholine receptor M3 protein (Peralta E.G. et al., EMBO J 6:3923-3929 (1987)), respectively.

These results suggest that the "BG2" proteins belong to the G protein-coupled receptor family, which further suggests that they participate in signal transduction through the activation of G proteins upon ligand binding.

Furthermore, the result of a northern blot analysis showed that the genes encoding the "BG2" proteins are specifically expressed in the brain. *In situ* hybridization detected a strong expression of the rat "BG2" gene in the hippocampus and spinal cord, and also in the hypothalamus, thalamus, and cerebellum.

The hippocampus plays an important role in memory and learning, the cerebellum regulates the body motions, and the hypothalamus serves as the center of the autonomic nervous system. Thus, the "BG2" proteins are assumed to be involved in the regulation of these functions. Therefore, the "BG2" proteins and genes, or an agonist or antagonist that can regulate the "BG2" protein function(s) can be used in the treatment of disabilities in memory and learning, or the control of the autonomous nervous system, such as regulation of, blood pressure, digestion, body temperature, and food-intake etc.

"BG2" protein may be prepared as natural protein, and also as recombinant protein by using recombinant DNA technology. A natural protein may be prepared, for instance, by extracting tissues such as the thalamus and hypothalamus, speculated to express "BG2" protein, and performing immunoaffinity chromatography using anti-"BG2" antibody as described later on. On the other hand, the recombinant protein can be prepared by culturing transformant cells carrying DNA encoding "BG2" protein as described later on.

One skilled in the art can prepare an altered protein having a function (transduction of intracellular signals through G protein activation) equivalent to that of the natural protein by introducing modifications such as replacement of any amino acid contained in the natural rat or human "BG2" protein (SEQ ID NO: 1, or SEQ ID NO: 20, respectively) according to known methods. Mutations of amino acids in a protein may occur naturally. The G protein-coupled receptor protein of the present invention includes such mutant proteins having an amino acid sequence altered by replacement, deletion or addition, having a function equivalent to that of the natural protein. The methods of altering amino acids, known to one skilled in the art, include, the Kunkel method (Kunkel T.A. et al., Methods Enzymol. 154:367-382 (1987)), double primer method (Zoller M.J. and Smith M., Methods Enzymol. 154:329-350 (1987)), cassette mutation (Wells et al., Gene 34:315-323 (1985)), and megaprimer method (Sarkar G. and Sommer S.S., Biotechniques 8:404-407 (1990)). The number of mutated amino acids in a functionally equivalent protein is generally not more than 10% of all the amino acids, favorably not more than 10 amino acids, and more favorably not more than 3 amino acids (for instance, one amino acid).

One skilled in the art can also use hybridization techniques (Hanahan D. and Meselson M., Methods Enzymol. 100:333-342 (1983); Benton W.D. and Davis R.W., Science 196:180-182 (1977)) to isolate a highly homologous gene from various other species based on the sequence of the rat or human "BG2" cDNA (SEQ ID NO: 2, or SEQ ID NO; 21, respectively) or a part of it, and use the isolated DNA to obtain a protein a functionally equivalent to these "BG2" proteins. Thus, it is possible for one skilled in the art to prepare a protein functionally equivalent to the rat or human "BG2" proteins, which is encoded by a DNA that hybridizes to the rat or human "BG2" cDNA. The G protein-coupled receptor protein of the present invention includes these proteins as well. Species used for isolation of a functionally equivalent protein include mouse, rabbit, sheep, bovine, dog, and pig, for instance, and particularly the brain tissues such as thalamus and hypothalamus are suitable.

DNA encoding a functionally equivalent protein to the rat or human "BG2" proteins has usually high homology to the nucleotide sequence of the rat or human "BG2" cDNA (SEQ ID NO: 2, and NO: 21). High homology generally means a sequence identity of not less than 70%, favorably not less than 80%, and more favorably not less than 90% at the nucleotide level. The sequence identity can be determined by the FASTA program.

Hybridization to isolate a DNA having high homology to the rat or human "BG2" cDNA is usually performed in "6x SSC, 40% formamide, at 25°C" followed by a wash in "1x SSC at 55°C," favorably in "6x SSC, 40% formamide, at 37°C" followed by a wash in "0.2x SSC at 55°C," and more favorably in "6x SSC, 50% formamide, at 37°C" followed by a wash in "0.1x SSC at 62°C."One skilled in the art may easily obtain a hybridization condition similar to the above conditions by appropriately selecting factors such as the dilution rate of SSC, the formamide concentration, and temperature.

The present invention also includes a partial peptide of the above-described G protein-coupled receptor protein. The partial peptide of the present invention includes, for instance, those corresponding to the N-terminal region of the G protein-coupled receptor protein, which can be utilized to prepare an antibody. The partial peptide of the invention has a length of at least 15 amino acids, and favorably 20 amino acids.

Furthermore, the present invention relates to a DNA encoding the G protein-coupled receptor protein of the invention as described above or its partial peptide. The DNA encoding the G protein-coupled receptor protein of the invention or its partial peptide includes cDNA, genomic DNA, and synthetic DNA, but is not limited as far as it encodes the protein or the peptide. cDNA encoding the G protein-coupled receptor of the invention can be obtained by screening a cDNA library from a tissue expressing the receptor (for instance, thalamus and hypothalamus) by using as a ³²P-labeled probe, the cDNA as described in SEQ ID NO: 2 or NO: 21, or a part of it, complementary RNA to the DNA, or a synthetic oligonucleotide comprising a part of the cDNA. Alternatively, cDNA may be cloned by synthesizing an oligonucleotide corresponding to the nucleotide sequence of the cDNA, and amplifying cDNA from an appropriate tissue (such as thalamus and hypothalamus) by PCR. Genomic DNA can be obtained by screening a genomic library by hybridization using as a ³²P-labled probe, the cDNA as described in SEQ ID NO: 2 or NO: 21, or a part of it, complementary RNA to the DNA, or a synthetic oligonucleotide comprising a part of the cDNA. Alternatively, it may be cloned by synthesizing an oligonucleotide corresponding to the nucleotide sequence of the cDNA, and amplifying genome DNA by PCR. Synthetic DNA can be prepared by chemically synthesizing oligonucleotides comprising a part of the nucleotide sequence of SEQ ID NO: 2 or NO: 21, annealing them into a double strand, and ligating them using DNA ligase (Khorana H.G. et al., J. Biol. Chem. 251:565-570 (1976); Goeddel D.V. et al., Proc. Natl. Acad. Sci. USA 76:106-110 (1979)).

These DNA can be used for producing recombinant proteins. Namely, it is possible to prepare the G protein-coupled receptor protein of the invention as a recombinant protein by inserting a DNA encoding the receptor protein (DNA as described in SEQ ID NO: 2 or NO: 21, for instance) into an appropriate expression vector, culturing a transformant obtained by introducing the vector into an appropriate cell, and purifying the expressed protein. Since the G protein-coupled receptor protein of the invention is a receptor protein, it is possible to prepare it in a form expressed on the cell membrane.

Specifically, if the host is *Escherichia coli*, the plasmid vectors such as pET-3 (Rosenburg A.H. et al., Gene 56:125-135 (1987)) and pGEX-1 (Smith D.B. and Johnson K.S., Gene 67:31-40 (1988)) may be used. *E.coli* can be transformed by the Hanahan method (Hanahan D., J. Mol. Biol. 166:557-580 (1983)), electroporation (Dower W.J. et al., Nucleic Acids Res. 16:6127-6145 (1988)), and such. If the host is fission yeast (Schizosaccharomyces pombe), a plasmid vector such as pESP-1 (Lu Q. et al., Gene 200:135-144 (1997)) is used. Yeast can be transformed by spheroplast fusion (Beach D. and Nurse P., Nature 290:140 (1981)), and lithium acetate method (Okazaki K. et al., Nucleic Acids Res. 18:6485-6489 (1990)), etc.

If the host is a mammalian cell, such as Chinese Hamster ovary-derived CHO cells and human HeLa cells, vectors such as pMSG (Clontech) are used. Recombinant DNA is introduced into mammalian cells by calcium phosphate method (Graham F.L. and van derEb A.J., J. Virology 52:456-467 (1973)), DEAE-dextran method (Sussman D.J. and Milman G., Mol. Cell. Biol. 4:1641-1643 (1984)), lipofection (Felgner P.L. et al., Proc. Natl. Acad. Sci. USA 84:7413-7417 (1987)), and electroporation (Neumann E. et al., EMBO J. 1:841-845 (1982)), etc. If the host is an insect cell, a baculovirus vector such as pBacPAK8/9 (Clontech) can be used. Transformation of insect cells is done by the methods described in the literature (BioTechnology 6:47-55 (1980)).

Recombinant proteins expressed in host cells can be purified by known methods. The proteins can also be synthesized as fusion proteins tagged with histidine residues at the N-terminus, or fused to glutathione-S-transferase (GST), and purified by using their binding ability toward a metal chelating resin, or a GST affinity resin (Smith M.C. et al., J. Biol. Chem. 263:7211-7215 (1988)), respectively. For instance, when the vector pESP-1 is used, the protein of interest is synthesized as a fusion protein with GST, which can be purified using GST affinity resin. The fusion protein may be digested with thrombin, or blood coagulating factor Xa to liberate the protein of interest.

Moreover, DNA encoding the G protein-coupled receptor protein of the present invention can be used in gene therapy of diseases that arise from a mutation of the protein. When used in gene therapy, the DNA can be introduced into human cells using retrovirus vectors (Danos O. and Mulligan R.C., Proc. Natl. Acad. Sci. USA 85:6460-6464 (1988); Dranoff et al., Proc. Natl. Acad. Sci. USA 90:3539-3543 (1993)), or adenovirus vectors (Wickham T.J. et al., Cell 73:309-319 (1993)), etc. To administer the vector to patients, transplantation of bone marrow, subcutaneous injection, and intravenous injection can be used (Asano S., Protein Nucleic acid and Enzyme 40:2491-2495 (1995)).

Furthermore, the present invention relates to an antibody that is capable of binding to the G protein-coupled receptor protein of the invention. Antibodies against the G protein-coupled receptor protein can be prepared by known methods in the art (for instance, refer to Shin-Seikagaku-Jikken-Kouza I: Protein I 389-406, Tokyo-Kagaku-Doujin). For instance, polyclonal antibodies are prepared as follows. An appropriate dose of the above protein or its partial peptide is administered into immune animals such as rabbits, guinea pigs, mice, or chickens. Administration may be performed together with the adjuvant (such as FIA or FCA) that promotes antibody production, and usually performed every couple of weeks. The titer of antibodies can be increased by performing multiple immunizations. After the final immunization, antisera are obtained by withdrawing blood from immune animals. Polyclonal antibodies are purified from antisera by ammonium sulfate precipitation, fractionation by anion exchange chromatography, or affinity chromatography with either Protein A or immobilized antigen. Monoclonal antibodies are prepared as follows. The G protein-coupled receptor proteins of the invention or its partial peptide is administered into immune animals as described above. After the final immunization, their spleens or lymph nodes are excised. Then, antigen-producing cells are recovered from the spleens or the lymph nodes, and fused with myeloma cells using polyethylene glycol etc. to produce hybridomas. Desired hybridomas are selected by screening, and their culture supernatant is used to prepare monoclonal antibodies. Monoclonal antibodies can be purified by ammonium sulfate precipitation, fractionation by anion exchange chromatography, or affinity chromatography with either Protein A or immobilized antigen. Antibodies prepared thereby can be used not only in affinity purification of the G protein-coupled receptor protein of the invention, but also for the diagnosis or antibody treatment of diseases arising from the abnormal expression of the receptors, or detection of the expression level of the receptors.

If used for antibody treatment, humanized antibodies or human antibodies are preferable. Humanized antibodies, in case of mouse-human chimeric antibodies, are prepared by isolating the gene encoding the antibody against the G protein-coupled receptor protein from the producing mouse cells, replacing the constant region of the H chain of the antibody with that of the human IgE, and introducing it into mouse myeloma J558L cells (Neuberger M.S. et al., Nature 314:268-270 (1985)). Human antibodies can be prepared by immunizing mice, whose immune system is replaced with that of human, with the G protein-coupled receptor protein.

Furthermore, the present invention relates to a method of screening ligands of the G protein-coupled receptor protein of the invention. The method includes such processes as exposing a test compound to the G protein-coupled receptor protein or its partial peptide, and selecting compounds that are capable of binding to the proteins or the peptide. Compounds to be tested include known compounds such as acetylcholine, adenosine, adrenaline, noradrenaline, angiotensin, bombesin, bradykinin, C5a, anaphylatoxin, calcitonin, cannabinoids, chemokines, cholecystokinin, dopamine, endothelin, formylmethionylpeptide, GABA, galanin, glucagon, glutamate, glycopeptide hormone, histamine, 5-hydroxytryptophan, leucotriene, melanocortin, neuropeptide Y, neurotensin, odorant, opioid peptide, opsin, parathyroid hormone, platelet activating factor, prostanoid, somatostatin, tachykinin, thrombin, thyrotropin releasing hormone, vasopressin, and oxytocin (Watson S. and Arkinstall S., G protein Linked Receptor FactsBook, Academic Press (1994)), and also other purified proteins, expressed products of genes (including libraries), extracts of tissues or cells in which the ligand is stipulated to be expressed (the brain, thalamus, and hypothalamus etc.), and the culture medium of the cells. The G protein-coupled receptor proteins may be used in a form expressed in desired cells (including transformants genetically engineered to express the proteins) or on the cell surface, in a form of the membrane fractions of the cells, or in a form bound to an affinity column. If necessary, test compounds may be labeled appropriately. Methods for labeling include radioisotope labeling, and fluorescence labeling, but not limited thereto. The binding between the G protein-coupled receptor proteins and test compounds can be examined by detecting the label added to the compound (for instance, measuring the radioactivity or fluorescence intensity), or using as an index, intracellular signaling triggered by the compound binding to the G protein-coupled receptor protein (such as G protein activation, the change in the concentration of Ca²⁺ or cAMP, phospholipase C activation, and the change in pH). Specific methods can be employed as described in the literatures (Cell Calcium 14:663-671 (1993); Analytical Biochemistry 226:349-354 (1995); J. Biol. Chem. 268:5957-5964 (1993); Cell 92:573-585 (1998); Nature 393:272-273 (1998)), and unexamined published Japanese patent application (JP-A) No. Hei 9-268). Alternatively, the binding may be detected by measuring the activity of the reporter gene using two-hybrid system (Zervos et al., Cell 72:223-232 (1994); Fritz et al., Nature 376:530-533 (1995)).

The present invention also relates to a method of screening for a compound which can inhibit the binding between the G protein-coupled receptor proteins of the invention and their ligands. The method includes processes of (a) exposing the ligand to the G protein-coupled receptors or their partial peptides in the presence of test compound, and detecting the binding ability between the ligand and the proteins or peptides, and (b) comparing the ability detected in (a) with that in the absence of the compound, and selecting compounds which are capable of decreasing the binding ability. Compounds to be tested include proteins, peptides, non-peptide compounds, artificially synthesized compounds, extracts of tissues and cells, sera, but not limited thereto. The G protein-coupled receptor proteins may be used in a form expressed in desired cells (including transformants genetically engineered to express the proteins) or on the cell surface, in a form of the membrane fractions of the cells, or in a form bound to an affinity column. If necessary, tested compounds may be labeled appropriately. Methods for labeling include radioisotope labeling, and fluorescence labeling, but not limited thereto. The binding between the G protein-coupled receptor proteins and test compounds can be examined by detecting the label added to the compound (for instance, measuring the radioactivity or fluorescence intensity), or using intracellular signaling, as an index, that are triggered by the compound binding to the G protein-coupled receptor protein (such as G protein activation, the change in the concentration of Ca2+ or cAMP, phospholipase C activation, and the change in pH). Specific methods can be employed as described in the literatures (Cell Calcium 14:663-671 (1993); Analytical Biochemistry 226:349-354 (1995); J. Biol. Chem. 268:5957-5964 (1993); Cell 92:573-585 (1998); Nature 393:272-273 (1998)), and JP-A Hei 9-268). If the result of the detection showed that the binding activity in the presence of a test compound is lower than that in the absence of the compound, the compound is judged to be capable of inhibiting the binding between the G protein-coupled receptors or their partial peptides and their ligands. These compounds include those capable of triggering the intracellular signaling through binding to the G protein-coupled receptor (agonist), and those not having such activity (antagonist). Agonists have similar bioactivities to those of the ligands of the G protein-coupled receptors. On the other hand, antagonists inhibit the bioactivities of the ligands. Therefore, these agonists and antagonists are useful as medicinal components for treatment of diseases arising from disorders in the signaling pathway mediated by the G protein-coupled receptors.

Furthermore, the present invention relates to a screening kit for compounds that inhibit the binding between the G protein-coupled receptor proteins and their ligands. The G protein-coupled receptor proteins or their partial peptides may be in a form expressed in desired cells (including transformants genetically engineered to express the protein) or on the cell surface, in a form of membrane fractions of the cell, or in a form bound to an affinity column. Components of the kit of the invention may include, other than the above described receptor protein samples, ligand samples (both labeled and unlabeled), and buffers for the reaction between the ligand and the receptor protein, and wash solutions. Labels to be added to the ligands include radioisotope and fluorescence, for instance. The kit of the invention can be used as described in JP-A Hei 9-268.

### Brief Description of the Drawings

Figure 1 shows the hydrophobicity plot of the mouse BG2 protein. The seven hydrophobic regions (transmembrane regions) that are characteristics of the G protein-coupled receptor proteins are indicated by the numbers from 1 to 7. The numbers in the bottom indicate those of the amino acid residues in the "BG2" protein.
Figure 2 shows the result of northern blot analysis of the tissue specific expression of the human and mouse BG2 genes.
Figure 3 shows the result of *in situ* hybridization analysis of the location of the mouse "BG2" gene expression in the brain.
Figure 4 shows the result of *in situ* hybridization analysis of the location of the mouse "BG2" gene expression in the spinal cord. "Sense" and "Antisense" indicate the results using sense RNA probe (not hybridizing with mRNA; negative control), and antisense RNA probe (hybridizing with mRNA), respectively.

### Best Mode for Carrying out the Invention

The present invention is illustrated in detail below with reference to examples, but is not to be construed as being limited thereto.

### Example 1: Isolation of a gene encoding a rat G protein-coupled receptor

The G protein-coupled receptors share a characteristic structure composed of seven transmembrane regions, and the amino acid sequences of the transmembrane regions and the adjacent regions are well conserved. The present inventors first compared the nucleotide sequences of the second and the seventh transmembrane domains, which are highly conserved, with known G protein-coupled receptors: mouse neuropeptide Y receptor Y1 (GenBank Accession Number Z18280), rat Y1 (Z11504), human Y1 (M84755), mouse neuropeptide Y receptor Y4 (U40189), rat Y4 (Z68180), human Y4 (Z66526), and mouse neuropeptide Y receptor Y6 (U58367), and synthesized novel sense and antisense primers, as described in SEQ ID NO: 3 and NO: 4, respectively.

Next, single stranded cDNA was synthesized from poly(A)⁺RNA prepared from rat thalamus and hypothalamus using the RNA-PCR kit (TaKaRa), and PCR was performed using the two primers. Specifically, poly(A)⁺RNA was prepared from rat thalamus and hypothalamus using Fasttrack 2.0 kit (Invitrogen). Then, 75 ng of the poly(A)⁺RNA was used to synthesize complementary DNA according to the protocol accompanying the RNA-PCR kit (TaKaRa). PCR amplification was performed using all the cDNA. The reaction mixture comprising each 0.15 mM dNTPs, 1.5 mM MgCl₂, 0.025 U/µl rTaq polymerase (TaKaRa), each 0.5 µM degenerated primer Fg (SEQ ID NO: 3) and Rb (SEQ ID NO: 4), and enzyme accompanying 10x PCR buffer was prepared making a total of 130 µl, and aliquoted into six 20 µl fractions. PCR was performed with the Pertier thermal cycler PTC200 (MJ Research) under conditions as follows: a single cycle of 94°C for 2 min, followed by 35 cycles of 94°C for 30 sec, 48°C for 1 min, 72°C for 1 min 30 sec, and then a single cycle of 72°C for 8 min. After PCR, the six reaction-solutions were combined, and the amplified products were purified using the Wizard PCR purification kit (Promega), and then eluted with 30 µl TE. 2 µl of the TE eluate was used for cloning into the pCR2.1 vector of the TOPO TA cloning kit (Invitrogen). XL1-Blue cells were used as the host cell and transformed using the *E.coli* pulser (BioRad). From the resulting transformants, 5,760 colonies having white or light blue color were randomly selected using the gene library construction system BioPick (BioRobotics), and inoculated into fifteen 384-well plates containing LB media supplemented with 100 µg/ml of ampicillin. Clones were cultured at 37°C overnight, and replica plated onto a filter on top of a LB agar plate containing 100 g/ml ampicillin and 25% glycerol, and another filter on top of a LB agar plate containing 100 µg/ml ampicillin, for preparing a glycerol stock, and colony hybridization, respectively, using a gene library replicating system BioGrid (BioRobotics).

Since the obtained PCR clones were expected to contain multiple overlapping clones of the NPY receptor cDNA, 80 clones out of the 5,760 clones were randomly selected, and their nucleotide sequences were partially determined. To determine the nucleotide sequence, plasmid DNA purified by the plasmid automatic isolating system PI100sigma (Kurabo) was used as a template. The sequence reactions were performed using the dye-primer-cycle sequencing kit FS (Perkin Elmer), and the reaction products were separated by electrophoresis using the DNA sequencer 377 (Perkin Elmer). The homology search of the obtained sequence using the blast program of the Wisconsin package (Genetic Computer Group) showed that 29 out of the 80 clones were the cDNAs encoding the coiled-coil like protein 1 (GeneBank Accession Number U79024) while 17 clones were those of the neuropeptide Y receptor Y1 (Z11504). Then, these two cDNA fragments were used as a probe for hybridization with the filters containing a library of the degenerated PCR amplified fragments. Probes were prepared by amplifying the insert of the respective clones by PCR, purifying the products using the Wizard PCR purification kit (Promega), and labeling them with [α-³²P] dCTP using the Prime-It II random primer labeling kit (Stratagene). Colony hybridization was performed according to the standard method (Sambrook et al., Molecular Cloning: A laboratory manual 2nd edition (1989)). Colonies that were negative for either the coiled-coil like protein 1 or the neuropeptide Y receptor Y1 were selected and their partial nucleotide sequences were determined. For DNA sequencing, the insert fragment of each clone was amplified by PCR from the culture medium, purified using the PCR product purification kit (Amersham), and used as a template. The sequence reactions were performed using the dye-primer-cycle sequencing kit FS (Perkin Elmer), and the reaction products were separated by electrophoresis using the DNA sequencer 377 (Perkin Elmer). The obtained sequences were analyzed by the homology search using the blast program of the Wisconsin package (Genetic Computer Group), and, as a result, a clone which has significant homology to the muscarinic acetylcholine receptor M5 (GeneBank Accession Number M22926) was found. The clone has been deposited at the National Institute of Bioscience and Human-Technology, Agency of Industrial Science and Technology.
Name of the depositary institution: National Institute of Bioscience and Human-Technology,Agency of Industrial Science and Technology, MITI.
Address of the depositary institution: 1-1-3 Higashi, Tsukuba, Ibaraki 305-8566, Japan.
Date of deposit: 12/25/1997.
Accession Number: FERM BP-6575

Next, in order to isolate the full-length cDNA of the gene, cDNA libraries were prepared from rat thalamus and hypothalamus. cDNA was synthesized according to the protocol accompanying the cDNA synthesizing kit (Stratagene), and the vector pEF1x and the host XL1-blue MRF' (Stratagene) were used. The pEF1x is a refined derivative of the pcDNA3 (Invitrogen) prepared as follows. (1) Preparation of the human EF1a promoter (GeneBank Accession Number J04617)

PCR was performed using human genomic DNA with primers (SEQ ID NO: 6/ CGAGGATCCGTGAGGCTCCGGTGCCCGTC; SEQ ID NO: 7/ CGGGTAAGCTTCACGACACCTGAAATGGAAGA). The products were digested with BamHI (TaKaRa) and HindIII (TaKaRa), and subcloned into the plasmid vector pUC19 (TaKaRa). The resulting plasmid was digested with XhoI, blunt-ended with the Klenow fragment (TaKaRa), and self-ligated using the DNA ligation kit (TaKaRa). The resulting plasmid was digested with BamHI and HindIII, and the insert was recovered.

### (2) Alteration of the pcDNA3

The pcDNA3 was digested with MluI (TaKaRa), blunt-ended with the Klenow fragment (TaKaRa), and self-ligated using the DNA ligation kit. The resulting plasmid was digested with AflIII (New England Biolabs) and SmaI (TaKaRa), blunt-ended with the Klenow fragment (TaKaRa), and self-ligated using the DNA ligation kit. Then, the obtained plasmid was digested with BglII (TaKaRa) and HindIII, and the fragment depleted of the CMV promoter was recovered, and ligated with the insert fragment as described in (1) using the DNA ligation kit to construct the pEF1x.

Next, the nucleotide sequence of the cDNA fragment was used to synthesize oligonucleotide probe (SEQ ID NO: 8/CCTTCTGCATCCCATTGTACGTACC), and multiple clones were obtained from the above cDNA libraries from rat thalamus and hypothalamus according to the protocol of the gene trapper cDNA positive selection system (GIBCO BRL). Then, colony hybridization was performed using the cDNA insert of the above isolated clone (FERM BP-6574) as a probe, and a positive clone was obtained. This clone has been deposited at the National Institute of Bioscience and Human-Technology, Agency of Industrial Science and Technology.
Name of the depositary institution: National Institute of Bioscience and Human-Technology, Agency of Industrial Science and Technology, MITI.
Address of the depositary institution: 1-1-3 Higashi, Tsukuba, Ibaraki 305-8566, Japan.
Date of deposit: 12/25/1997.
Accession Number: FERM BP-6574

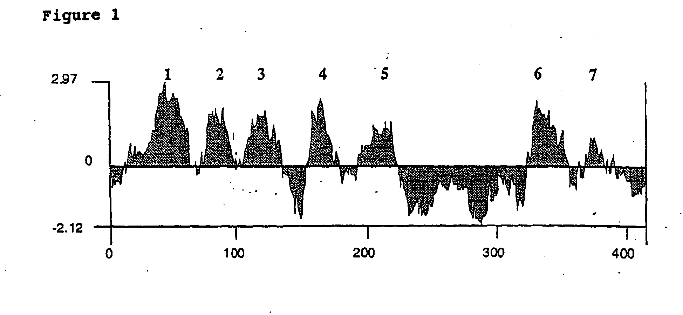

The insert fragment of the clone was of 2.7 kb. Plasmid DNA was prepared by the QIAprep Midi Kit (QIAGEN), and the complete nucleotide sequence was determined using the shotgun cloning method (Sambrook et al., Molecular Cloning: A laboratory manual 2nd edition (1989)). cDNA fragmentation was performed using the closed sonifier biomaterial treating system Biorupter (Tousou Denki), and the DNA fragments were separated by electrophoresis on a 2% agarose gel. Fragments of around 0.6 kb were purified using the gene clean spin kit (bio101), blunt-ended with T4 DNA polymerase (TaKaRa), and cloned into the HincII-BAP treated pUC118 vector, XL1-Blue was used as a host cell, and transformed using the *E.coli* pulser (BioRad). The obtained shotgun clones were sequenced using the dye-primer cycle sequencing kit FS (Perkin Elmer), or the dye-terminator cycle sequencing kit (Perkin Elmer). The resulting sequences were combined and edited to get the complete nucleotide sequence using the DNA sequencing software Sequencher (Hitachi Software). The complete nucleotide sequence is composed of 2700 bp, and turned out to be encoding a protein of 413 amino acids (SEQ ID NO: 5). Because there is a stop codon in the 5' region of the open reading frame, the cDNA is supposed to include the entire coding region (SEQ ID NO: 2). When this sequence was translated into the amino acid sequence, the hydrophobicity plot identified seven transmembrane regions from 1 to 7 (Figure 1).

In addition, the open reading frame size was approximately 1.2 kb, which is similar to that of the known G protein-coupled receptors. G protein-coupled receptor proteins have common features in their amino acid sequences, and thus form a protein family. As a result of the homology search using the amino acid sequence encoded by the isolated cDNA, the encoded protein was found to be a novel receptor protein having a homology of 26%, 25%, and 29% to known G protein-coupled receptors: bovine muscarinic acetylcholine receptor M3 protein (Lee P.H. et al., Biochim. Biophys. Acta 1223:151-154 (1994)), human muscarinic acetylcholine receptor M5 protein (Bonner T.I. et al., Neuron 1:403-410 (1988)), and mouse a2A adrenoreceptor (Link R. et al., Mol. Pharmacol. 42:16-27 (1992)), respectively.

### Example 2: The isolation of the human G protein-coupled receptor gene

The obtained rat sequence was subjected to EST search to reveal a fragment of the human homologue (gene bank NID: 946030 and NID: 901756). Human fetal brain cDNA was amplified by PCR using the specific primers IF01 (SEQ ID NO: 9/ CTTCCGCCGGGCCTTCACCAA) and IR02 (SEQ ID NO: 10/ ACAGACACGGCGGGGCTCAC) (probe 1). A human λ EMBL3 SP6/T7 genomic library (Clontech) of a size of 1.2x10⁶ pfu was screened using probe 1 according to standard procedure plaque hybridization procedures. Two positive clones were thus isolated. The obtained phage-clones were digested with SacI, and three bands of a clone were subcloned. These fragments, termed I1 (SEQ ID No: 11), I3 (SEQ ID No: 12) and I5 (SEQ ID No: 13), were sequenced and a hypothetical sequence was speculated by comparing with the rat homologue. I1 and I3 were subjected to PCR amplification using specific primers YS03 (SEQ ID NO: 14/ TGAACGCTTCGGGGGCGCTG) and YS05 (SEQ ID NO: 15/ GAGATGGCGAGGTTGAGCAGG), YS12 (SEQ ID NO: 16/GGCTCCAAGCCATCGGCGTC) and YS14(SEQ ID NO: 17/CTCACTTCCAGCAGTGCTCC) and the PCR products were termed probe 2 and probe 3, respectively. Human hypothalamus cDNA (1.3 x 10⁶ phage) was plated at a density of 5.6 x 10⁴ pfu/150mm plate. The obtained sub-pools were checked by PCR using the primers YS03 and YS05. One positive sub-clone was screened in the same method as the screening of the genomic library, using probe 2. One cDNA clone containing 5'UTR to TM5 was obtained and named cDNA clone 1.

Probe 4 was amplified by PCR from cDNA clone 1 using the primers YS07 (SEQ ID NO: 18/ GCCTCCGCACCCAGAACAAC) and YS10 (SEQ ID NO: 19/ TGCGCCTCTGGATGTTCAG). Phase screening of the human hippocampus library (3x10⁶ pfu) was done in the same method as the genomic library, using probe 3 and probe 4. A few clones were obtained and the longest one, termed cDNA clone 2, was sequenced. It has the region between TM2 and 3'UTR. cDNA clone 1 was digested by SacII, and the 3.3kb band, which contained vector and the 5'-end region, was treated by shrimp alkaline phosphatase. cDNA clone 2 was also digested by SacII, and the 1.7kb fragment was ligated into the 3.3kb fragment from cDNA clone 1. The clone into which this ligated fragment was inserted has been deposited at the National Institute of Bioscience and Human-Technology, Agency of Industrial Science and Technology.
Name of the depositary institution: National Institute of Bioscience and Human-Technology, Agency of Industrial Science and Technology, MITI.
Address of the depositary institution: 1-1-3 Higashi, Tsukuba, Ibaraki 305-8566, Japan.
Date of deposit: 12/17/1998.
Accession Number: FERM BP-6609

Human "BG2" cDNA nucleotide sequence is shown in SEQ ID NO: 21, and the amino acid sequence of the protein encoded by the said cDNA in SEQ ID NO: 20.

Human "BG2" protein had 32%, 28%, and 27% homology to known G protein-coupled receptors; human α-2C-1 adrenoreceptor (Regan J.W. et al., Proc. Natl. Acad. Sci. USA 85:6301-6305 (1988)), mouse β-1 adrenoreceptor (Jasper J.R. et al., Biochim. Biophys. Acta 1178:307-309 (1993)), and human muscarinic acetylcholine receptor M3 protein (Peralta E.G. et al., EMBO J. 6:3923-3929 (1987)), respectively.

### Example 3: Northern blot analysis

Probe 4 was labeled with ³²Pγ-dCTP (Amersham, Prime It II) and used as cDNA probe for the detection of human "BG2". Human Multiple Tissue Northern (MTN) Blots Membrane with Express HybriTM was purchased from Clontech. After prehybridization of the membrane at 68°C for 30 min, it was hybridized with the probe at 68°C for 1 hr (final concentration of the probe was 1.5 x 10⁶cpm/ml.) The blot was rinsed with 2 x SSC containing 01% SDS at 42°C for 30 min, and the final wash was done at 50°C for 30 min in 0.1 x SSC containing 0.1% SDS. The blot was then exposed at -80°C for 2.5 days to Kodak autoradiographic film.

For the detection of the mouse "BG2," probe was prepared by PCR amplifying using the rat "BG2" cDNA as a template with sense primer MF2 (SEQ ID NO: 22/ TGCATCCCATTGTACGTNCC), and antisense primer MR1 (SEQ ID NO: 24/ TGCTCTGGGACACCATCTTC), purifying the amplified products by electrophoresis on an agarose gel, and labeling them as above described for human gene.

Blotting membrane used was the Rat MTN (Multiple Tissue Northern) blot (Clontech). Hybridization was performed at 42°C overnight in hybridization buffer (50% formamide, 4x SSPE, 1% SDS, 0.5% BLOTTO, and 100 µg/ml salmon sperm DNA). The membrane was washed at 65°C in 0.1x SSC containing 0.1% SDS, and then exposed to the Kodak autoradiography film at -80°C overnight.

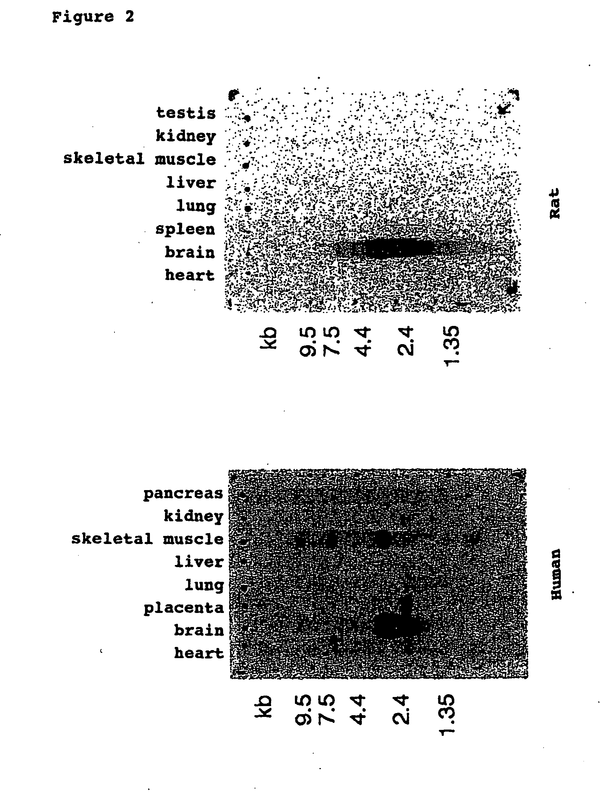

The result showed that the human and rat "BG2" genes are strongly expressed particularly in the brain (Fig. 2).

### Example 4: In situ Hybridization

Adult male Sprague-Dawley rats (Charles River Japan) aged 13-18 weeks were anesthetized with inhalation of ether, connected to a rotary pump and infused with chilled 4% paraformaldehyde in phosphate buffer (pH7.2) via a cannula inserted into the left ventricle. After perfusion, brain, pituitary and spinal cord were removed and dissected to sagittal or coronal sections. The tissue specimens were postfixed with the same fixative overnight at 4°C. The following process was carefully done to avoid RNA contamination. Tissue specimens were embedded in paraffin wax in a routine manner, then paraffin sections were cut into a thickness of 6 µm by rotary microtome (Model HM 355; MICROM Laborgerate GmbH). The sections were stored in moisture free condition at -20°C until proceeded to *in situ* hybridization.

For preparation of rat BG2 sense and antisense RNA probes, the cDNA fragment, amplified by PCR from MP-21 plasmid DNA using a sense primer MF2 (SEQ ID NO:22/ TGCATCCCATTGTACGTNCC) and antisense primer MR3 (SEQ ID NO:23/ ATCATTAGGAGCGTGTANGG), was cloned into pZErO-2 vector (Invitrogen). The RNA probes were labeled with digoxigenin using DIG RNA Labeling Kit (Boehringer Mannheim). Paraffin sections were de-paraffinized with xylene and transferred to distilled water after rinsing with graded ethyl alcohol. *In situ* Hybridization Reagents (ISHR, Code No. 316-01951; Nippon Gene) were used as reagents without digoxigenin-labeled RNA. The sections were incubated with two changes of phosphate buffer saline(PBS;ISHR 1) for 1 min and 10 min. The sections were treated with proteinase K (ISHR 6) for 10 min at 37°C . Acetylation was done with acetylation buffer (ISHR 3) containing acetic anhydride (ISHR 4) for 15 min, followed by quenching with PBS/glycine buffer (ISHR 2) for 20 min at room temperature, rinsed twice with 4 x SSC (ISHR 5) for 10 min and then rinsed with PBS buffer for 10 min. After pre-hybridization with 50% formamide/2 x SSC for 30 min at room temperature, hybridization was performed for 16 hr at 42 °C using digoxigenin-labeled RNA probe (1 µg/ml).

Post hybridization washing was performed twice 50% formamide/2 x SSC for 10 min at 42°C . Then the sections were treated with RNase A (ISHR10)/NET buffer (ISHR 9) for 30 min at 37°C after rinsing with NET buffer (ISHR 9) for 5 min at 37°C. After washing twice with 0.1 x SSC buffer (ISHR 11) for 20 min, the sections were transferred and labeled digoxigenin was detected using the Digoxigenin Detection Kit (Boehringer Manheim). Then the sections were rinsed with 100 mM Tris-Hcl, 150 mM NaCl containing buffer (Buffer 1) for 1 min at room temperature then incubated with blocking reagent (Buffer 2) for 30 min at room temperature. The sections were incubated with anti-digoxigenin alkaline phosphatase-labeled antibody for 60 min at room temperature. After washing with Buffer 1 for 15 min and Buffer 3 for 2 min at room temperature, the solutions were incubated with NBT/X-phosphate solution diluted with Buffer 3 for 12-14 hr at room temperature. The sections were mounted with glycerol or Permount after washing with Buffer 4.

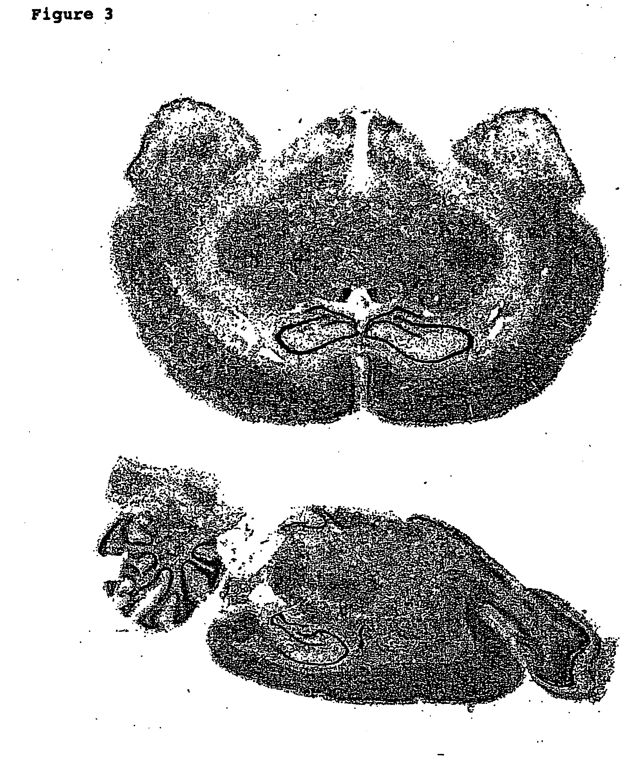

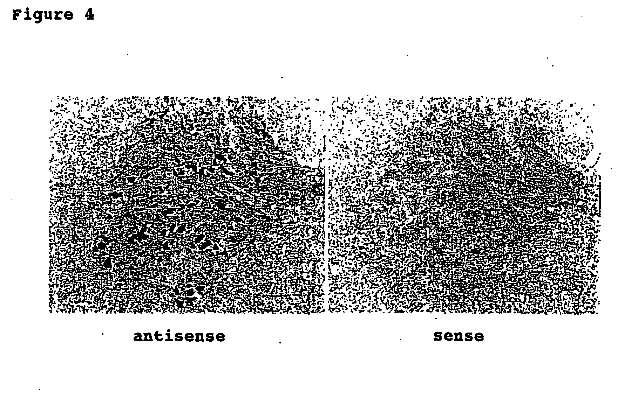

As a result, as shown in Figures 3 and 4, BG2 cDNA probe was strongly hybridized to the hippocampus and the spinal cord. Hybridization of a medium extent was also detected in hypothalamus and cerebellum.

### Industrial Applicability

The present invention has provided novel G protein-coupled receptor proteins specifically expressed in the brain, and their genes. Use of the receptors makes it possible to screen their ligands and compounds that are candidates for medicines. These ligands and candidate compounds would be useful in the diagnosis and treatment of diseases arising from disorders of signal transduction pathway mediated by the G protein-coupled receptor of the invention.

## Claims

1. A DNA encoding a guanosine triphosphate binding protein-coupled receptor protein selected from the group consisting of
(a) a DNA comprising the nucleotide sequence of SEQ ID NO:2 or 21;
(b) a DNA encoding a polypeptide comprising the amino acid sequence of SEQ ID NO:1 or 20;
(c) a DNA with a sequence identity of not less than 90% to the entire nucleotide sequence of the DNA of SEQ ID NO:2 or 21 or a DNA as defined in (b); and
(d) a DNA encoding a polypeptide having the amino acid sequence of (a) or (b), wherein one to three amino acids are replaced, deleted or added.

2. A vector comprising the DNA of claim 1.

3. A transformant carrying the vector of claim 2.

4. A method of producing a guanosine triphosphate binding protein-coupled receptor protein comprising culturing the transformant of claim 3.

5. A polypeptide encoded by the DNA of claim 1 or obtainable by the process of claim 4.

6. An antibody which binds to the polypeptide of claim 5.

7. A method of screening for a ligand of the polypeptide of claim 5, the method comprising exposing a test compound to the polypeptide of claim 5 and selecting a compound that binds to said polypeptide.

8. A method of screening for a compound that inhibits the binding between the polypeptide of claim 5 and its ligand, the method comprising
(a) exposing a ligand to the polypeptide of claim 5 in the presence of a test compound and detecting the binding activity between the polypeptide of claim 5 and the ligand; and
(b) comparing the binding activity detected in (a) with that in the absence of the test compound, and selecting a compound that reduces the binding activity between the polypeptide of claim 5 and the ligand.

9. A kit for screening a compound that inhibits the binding between the polypeptide of claim 5 and the ligand, the kit comprising the polypeptide of claim 5.

10. A pharmaceutical composition comprising the DNA of claim 1, the polypeptide of claim 5 or the antibody of claim 6.

11. Use of the DNA of claim 1 or the antibody of claim 6 for the preparation of a pharmaceutical composition for the treatment of diseases arising from the abnormal expression of a guanosine triphosphate binding protein-coupled receptors or diseases that arise from a mutation in said receptor.

12. Use of the polypeptide of claim 5 for the preparation of a pharmaceutical composition for the treatment of disabilities in memory and learning, diseases relating to the regulation of blood pressure, digestion, body temperature or food intake or other diseases relating to the control of the autonomous nervous system.

13. The DNA of claim 1 or the antibody of claim 6 for use in the treatment of diseases arising from the abnormal expression of a guanosine triphosphate binding protein-coupled receptors or diseases that arise from a mutation in said receptor.

14. The polypeptide of claim 5 for use in the treatment of disabilities in memory and leaning, diseases relating to the regulation of blood pressure, digestion, body temperature or food intake or other diseases relating to the control of the autonomous nervous system.

## Patentansprüche

1. DNA, die ein an ein Guanosintriphosphat bindendes Protein gekoppeltes Rezeptorprotein codiert, ausgewählt aus der Gruppe bestehend aus
(a) einer DNA, die die Nucleotidsequenz von SEQ ID NO:2 oder 21 umfasst;
(b) einer DNA, die ein Polypeptid codiert, das die Aminosäuresequenz von SEQ ID NO:1 oder 20 umfasst;
(c) einer DNA mit einer Sequenzidentität von nicht weniger als 90% zur gesamten Nucleotidsequenz der DNA von SEQ ID NO:2 oder 21 oder einer DNA wie in (b) definiert; und
(d) einer DNA, die ein Polypeptid codiert, das die Aminosäuresequenz von (a) oder (b) aufweist, wobei ein bis drei Aminosäuren ersetzt, deletiert oder hinzugefügt sind.

2. Vektor, der die DNA nach Anspruch 1 umfasst.

3. Transformante, die den Vektor nach Anspruch 2 trägt.

4. Verfahren zur Herstellung eines Rezeptorproteins, das an ein Guanosintriphosphat bindendes Protein gekoppelt ist, umfassend das Züchten der Transformante nach Anspruch 3.

5. Polypeptid, das von der DNA nach Anspruch 1 codiert wird oder durch das Verfahren nach Anspruch 4 erhältlich ist.

6. Antikörper, der an das Polypeptid nach Anspruch 5 bindet.

7. Verfahren zum Screenen nach einem Liganden des Polypeptids nach Anspruch 5, wobei das Verfahren das Aussetzen einer Testverbindung gegenüber dem Polypeptid nach Anspruch 5 und das Auswählen einer Verbindung umfasst, die an das Polypeptid bindet.

8. Verfahren zum Screenen nach einer Verbindung, die die Bindung zwischen dem Polypeptid nach Anspruch 5 und seinem Liganden inhibiert, wobei das Verfahren umfasst
(a) Aussetzen eines Liganden gegenüber dem Polypeptid nach Anspruch 5 in Anwesenheit einer Testverbindung und Nachweisen der Bindungsaktivität zwischen dem Polypeptid nach Anspruch 5 und dem Liganden; und
(b) Vergleichen der in (a) nachgewiesenen Bindungsaktiviät mit jener in Abwesenheit der Testverbindung und Auswählen einer Verbindung, die die Bindungsaktivität zwischen dem Polypeptid nach Anspruch 5 und dem Liganden reduziert.

9. Kit zum Screenen einer Verbindung, die die Bindung zwischen dem Polypeptid nach Anspruch 5 und dem Liganden inhibiert, wobei der Kit das Polypeptid nach Anspruch 5 umfasst.

10. Arzneimittel, das die DNA nach Anspruch 1, das Polypeptid nach Anspruch 5 oder den Antikörper nach Anspruch 6 umfasst.

11. Verwendung der DNA nach Anspruch 1 oder des Antikörpers nach Anspruch 6 zur Herstellung eines Arzneimittels für die Behandlung von Erkrankungen, die aus abnormer Expression eines Rezeptorproteins, das an ein Guanosintriphosphat bindendes Protein gekoppelt ist, resultieren oder von Erkrankungen, die aus einer Mutation in dem Rezeptor resultieren.

12. Verwendung des Polypeptids nach Anspruch 5 zur Herstellung eines Arzneimittels für die Behandlung von Gedächtnis- und Lernstörungen, Erkrankungen, die die Regulierung von Blutdruck, Verdauung, Körpertemperatur oder Nahrungsaufnahme betreffen, oder von anderen Erkrankungen, die die Kontrolle des autonomen Nervensystems betreffen.

13. DNA nach Anspruch 1 oder Antikörper nach Anspruch 6 zur Verwendung bei der Behandlung von Erkrankungen, die aus der abnormen Expression eines Rezeptorproteins, das an ein Guanosintriphosphat bindendes Protein gekoppelt ist, resultieren oder von Erkrankungen, die aus einer Mutation in dem Rezeptor resultieren.

14. Polypeptid nach Anspruch 5 zur Verwendung bei der Behandlung von Gedächtnis- und Lernstörungen, Erkrankungen, die die Regulierung von Blutdruck, Verdauung, Körpertemperatur oder Nahrungsaufnahme betreffen, oder von anderen Erkrankungen, die die Kontrolle des autonomen Nervensystems betreffen.

## Revendications

1. ADN codant une protéine récepteur couplée à une protéine liant la guanosine triphosphate sélectionné parmi le groupe consistant en
(a) un ADN comprenant la séquence nucléotidique de la SEQ ID NO : 2 ou 21 ;
(b) un ADN codant un polypeptide comprenant la séquence d'acides aminés de la SEQ ID NO : 1 ou 20 ;
(c) un ADN ayant une identité de séquence de pas moins que 90 % par rapport à la séquence nucléotidique entière de l'ADN de la SEQ ID NO : 2 ou 21 ou d'un ADN tel que défini dans (b) ; et
(d) un ADN codant un polypeptide ayant une séquence d'acides aminés de (a) ou (b), dans laquelle un à trois acides aminés sont remplacés, supprimés ou ajoutés.

2. Vecteur comprenant un ADN selon la revendication 1.

3. Transformant portant le vecteur selon la revendication 2.

4. Méthode de production d'une protéine récepteur couplée à une protéine liant la guanosine triphosphate comprenant la culture du transformant selon la revendication 3.

5. Polypeptide codé par un ADN selon la revendication 1 ou susceptible d'être obtenu par le procédé selon la revendication 4.

6. Anticorps qui se lie au polypeptide selon la revendication 5.

7. Méthode de criblage d'un ligand du polypeptide selon la revendication 5, la méthode comprenant l'exposition d'un composé test au polypeptide selon la revendication 5 et la sélection d'un composé qui se lie audit polypeptide.

8. Méthode de criblage d'un composé qui inhibe la liaison entre le polypeptide selon la revendication 5 et son ligand, la méthode comprenant
(a) l'exposition d'un ligand au polypeptide selon la revendication 5 en présence d'un composé test et la détection de l'activité de liaison entre le polypeptide selon la revendication 5 et le ligand ; et
(b) la comparaison de l'activité de liaison détectée en (a) avec celle en absence du composé test, et la sélection d'un composé qui diminue l'activité de liaison entre le polypeptide selon la revendication 5 et le ligand.

9. Kit de criblage d'un composé qui inhibe la liaison entre le polypeptide selon la revendication 5 et le ligand, le kit comprenant le polypeptide selon la revendication 5.

10. Composition pharmaceutique comprenant un ADN selon la revendication 1, un polypeptide selon la revendication 5 ou un anticorps selon la revendication 6.

11. Utilisation d'un ADN selon la revendication 1 ou d'un anticorps selon la revendication 6 pour la préparation d'une composition pharmaceutique destinée au traitement de maladies résultant d'une expression anormale de récepteurs couplés à une protéine liant la guanosine triphosphate ou de maladies résultant d'une mutation dudit récepteur.

12. Utilisation d'un polypeptide selon la revendication 5 pour la préparation d'une composition pharmaceutique destinée au traitement de troubles de la mémoire ou de l'apprentissage, de maladies liées à la régulation de la pression sanguine, à la digestion, à la température corporelle ou à la prise alimentaire ou d'autres maladies liées au contrôle du système nerveux autonome.

13. ADN selon la revendication 1 ou anticorps selon la revendication 6 pour une utilisation dans le traitement de maladies résultant d'une expression anormale de récepteurs couplés à une protéine liant la guanosine triphosphate ou de maladies résultant d'une mutation dudit récepteur.

14. Polypeptide selon la revendication 5 pour une utilisation dans le traitement de troubles de la mémoire ou de l'apprentissage, de maladies liées à la régulation de la pression sanguine, à la digestion, à la température corporelle ou à la prise alimentaire ou d'autres maladies liées au contrôle du système nerveux autonome.
